Europäisches Patentamt

European Patent Office

Office européen des brevets

(19)

(11) Numéro de publication : **0 080 956**
**B1**

(12)

# FASCICULE DE BREVET EUROPÉEN

(45) Date de publication du fascicule du brevet :
28.08.85

(21) Numéro de dépôt : **82420162.8**

(22) Date de dépôt : **25.11.82**

(51) Int. Cl.⁴ : **C 07 K   3/02, C 07 K   3/28,
C 07 K  15/06, C 08 L  89/06,
A 61 L  17/00, A 61 F   2/00**

(54) Procédé de préparation industrielle de matériaux collagéniques à partir de tissus placentaires humains, matériaux collagéniques humains obtenus, leur application comme biomatériaux.

(30) Priorité : 26.11.81 FR 8122606

(43) Date de publication de la demande :
08.06.83 Bulletin 83/23

(45) Mention de la délivrance du brevet :
28.08.85 Bulletin 85/35

(84) Etats contractants désignés :
BE CH DE GB IT LI NL SE

(56) Documents cités :
DIE ANGEWANDTE MAKROMOLEKULARE CHEMIE,
vol. 82, no. 1276, 1979, pages 171-186, Hüthig & Wepf
Verlag, Berlin (DE); R. RIEMSCHNEIDER et al.:
"Pepsin-solubilized collagen from cow placenta".
HOPPE-SEYLER'S Z. PHYSIOL. CHEM., vol. 356, mai
1975, pages 567-575; W. HENKEL: "Isolierung und
Eigenschaften eines nach Alkali-Vorbehandlung
löslichen Arterienkollagens".
BIOCHEMISTRY, vol. 18, no. 14, 1979, pages 3089-
3097; T.F. KRESINA et al.: "Isolation and characterization of basement membrane collagen from human
placental tissue. Evidence for the presence of two
genetically distinct collagen chains".
CHEMICAL ABSTRACTS, vol. 85, no. 15, 11 octobre
1976, page 208, no. 105644w, Columbus Ohio (USA);
L.P. ISTRANOV et al.: "Dispersion of optical rotation
and circular dichroism of enzyme- and alkali-dissolved collagen".

(73) Titulaire : **FONDATION MERIEUX**
**17, rue Bourgelat**
**F-69002 LYON (FR)**

**CENTRE TECHNIQUE DU CUIR**
**181, avenue Jean Jaurès**
**F-69007 Lyon (FR)**

(72) Inventeur : **Bonneau, Marc**
**42 rue Bélissen**
**Lyon 5ème, Rhône (FR)**
Inventeur : **Play, Dominique**
**2, rue de Gerland**
**Lyon 7ème, Rhône (FR)**
Inventeur : **Herbage, Daniel**
**31, quai Saint-Vincent**
**Lyon 1er, Rhône (FR)**
Inventeur : **Merieux, Charles**
**17, boulevard des Belges**
**Lyon 6ème, Rhône (FR)**
Inventeur : **Comte, Philippe**
**Le Géant 47, avenue Valioud**
**Sainte-Foy-les-Lyon, Rhône (FR)**

(74) Mandataire : **Maureau, Pierre et al**
**Cabinet GERMAIN & MAUREAU Le Britannia - Tour C**
**20, Boulevard E. Déruelle**
**F-69003 Lyon (FR)**

## Description

La présente invention concerne un procédé de préparation industrielle de nouveaux matériaux collagéniques, insolubles et solubles, à partir de tissus placentaires humains ainsi que les matériaux collagéniques humains obtenus et leur application comme biomatériaux.

Les collagènes sont les principaux constituants protéiniques de tissus conjonctifs comme le derme, le cartilage, les tendons, les parois vasculaires... auxquels ils apportent les propriétés mécaniques essentielles à leurs fonctions physiologiques. Actuellement au moins cinq types principaux de collagènes ont été isolés et caractérisés. Ils peuvent être classés en deux groupes principaux : les collagènes de types I, II, III, présents dans les tissus sous forme de fibres et souvent appelés collagènes intersticiels et les collagènes de types IV et V appelés collagènes de membranes basales dont la composition moléculaire et la forme au sein des tissus sont encore mal connues.

Ces collagènes, bien que différents de par leur séquence primaire, possèdent une structure tertiaire en triple hélice commune, structure qui les protège contre la dégradation par les enzymes protéolytiques autres que les collagénases.

Après leur synthèse intracellulaire, les collagènes sont excrétés sous une forme soluble dans le milieu extracellulaire où ils s'aggrègent pour former des fibrilles. Au cours de la maturation des tissus, ces fibrilles s'insolubilisent rapidement du fait de la réticulation des molécules de collagènes par la formation de liaisons de covalence interchaines. Ces liaisons prennent naissance à l'extrémité d'au moins une des molécules dans une région non hélicoïdale donc dans une région susceptible d'être clivée par l'action d'enzymes protéolytiques.

Deux types de procédés permettent actuellement d'extraire d'un tissu des quantités notables de collagènes.

Un de ces procédés utilise des tissus de jeunes mammifères dans lesquels existe une fraction importante de collagène non réticulé et utilise les propriétés de solubilité de ces collagènes dans des solutions salines neutres ou dans des acides dilués comme décrit par exemple dans les brevets français 1 568 829 et 1 596 789 qui préparent du collagène soluble à partir de la peau de veau.

Ces procédés d'extraction présentent toutefois plusieurs inconvénients : d'une part ils nécessitent l'utilisation comme matière de base, des seuls tissus animaux et d'autre part, ils ne permettent d'obtenir que du collagène type I.

D'autres types de procédés utilisent des tissus matures ; il devient alors indispensable d'utiliser l'activité protéolytique d'enzymes comme la pepsine, la pronase ou la trypsine pour couper les télopeptides des collagènes non protégés par la triple hélice et rompre ainsi des liaisons intermoléculaires responsables de leur insolubilité.

Parmi les tissus humains, seul le tissu placentaire est disponible en quantité suffisante pour permettre éventuellement une préparation de collagènes humains à l'échelle industrielle.

Les difficultés rencontrées pour la préparation de collagènes purifiés à partir de ce tissu sont nombreuses et proviennent essentiellement :
— de la faible teneur en collagènes du tissu de départ (de 10 à 15 %)
— de l'insolubilité de ces collagènes
— de la présence possible dans le matériau de départ des virus de l'hépatite.

On a proposé de traiter ce tissu par des solutions salines (ClNa) neutres ou acides (demande de brevet allemand DAS 26 16 939) ou des solutions d'urée concentrées (demande de brevet allemand OS 24 62 221) afin d'en extraire la faible partie soluble. Différents travaux effectués au niveau laboratoire ont amélioré cette extraction des collagènes à partir de tissu placentaire par digestion ménagée à la pepsine de 20 à 40 % des collagènes de départ (cf. en particulier BIOCHEMISTRY, vol. 18, n° 14, 10 juillet 1979, T. F. Kresina, p. 3091) et leur fractionnement en collagènes de types I, III, IV, V. Les prinipaux inconvénients de ces méthodes sont les suivants :
— la digestion enzymatique du tissu total nécessite le traitement de poids et de volume importants de tissu et d'extraits et l'utilisation de quantités élevées d'enzymes. En effet, l'enzyme protéolytique agit à ce niveau non seulement sur les télopeptides du collagène mais aussi sur la masse des protéines non collagéniques (60 % du poids sec de départ)
— il n'est possible d'extraire que 20 à 40 % du collagène total et les extraits sont très peu concentrés en collagène
— les virus de l'hépatite contaminent souvent les différentes fractions extraites et donc empêchent l'utilisation des produits comme biomatériaux. Il devient alors nécessaire de partir de tissus placentaires préalablement sélectionnés par une méthode immunologique et dépourvus de ce contaminant viral.

On a d'autre part proposé (Die angewandte Makromolekulare Chemie, vol. 82 n° 1276, 1979, p. 174, tableau 1) de prétraiter du placenta de vache avec une solution diluée de soude ou d'acétate de sodium (0,1 M) ; ce prétraitement en milieu aussi faiblement alcalin ne permet toutefois d'extraire, au bout de 24 heures, que 0,5 % de collagène ; il n'augmente pas d'autre part le taux de solubilisation ultérieur du collagène par action de la pepsine, taux qui reste aux environs de 30 %.

On a également proposé (Hoppe-Seyler's Z. Physiol. Chem. Bd 356 pp. 567-575, mai 1975) de traiter des fragments d'aorte à température ambiante pendant une durée pouvant atteindre cinq jours dans un milieu fortement alcalin afin d'obtenir du collagène I.

La présente invention s'est donnée pour but de remédier à ces inconvénients en proposant un procédé de traitement industriel de tissus placentaires humains qui permet d'obtenir de nouveaux matériaux collagéniques insolubles ainsi que des collagènes solubles de différents types notamment dépourvus de contamination virale.

Ce procédé présente l'originalité :

— de partir de lots de tissus placentaires humains même contaminés par les virus de l'hépatite ;

— de solubiliser jusqu'à 40 % du collagène total sans avoir à utiliser des enzymes protéolytiques ;

— de solubiliser la quasi totalité du collagène par une attaque enzymatique en utilisant de très faibles quantités d'une large gamme d'enzymes protéolytiques ;

— d'obtenir des collagènes légèrement modifiés (désamidation et élimination d'une partir des télopeptides), mais conservant leurs propriétés biologiques ;

— de préparer un matériau en collagène insoluble pratiquement pur utilisable comme support de cultures de différents types de cellules ;

— tous les composés obtenus étant dépourvus de contamination virale peuvent être utilisés comme biomatériaux.

L'invention concerne un procédé de préparation industrielle de matériaux collagéniques à partir de tissus placentaires, comportant une étape de prétraitement desdits tissus, un traitement en milieu alcalin desdits tissus et un traitement éventuel de digestion de résidus insolubles, issus du traitement alcalin, caractérisé en ce qu'on part de tissus placentaires humains, on prétraite ces tissus par pressage, broyage et au moins un lavage en milieu neutre ou acide, on soumet les tissus ainsi prétraités à un milieu fortement alcalin à une température inférieure ou égale à 10 °C jusqu'à obtention de portions de collagène solubilisées et d'un résidu insoluble, et on soumet à une chromatographie sur résine échangeuse d'anions, à une température inférieure ou égale à 10 °C suivie d'une précipitation fractionnée des collagènes par des sels en milieu acide, lesdites portions de collagène solubilisées ainsi que, éventuellement, les solutions de collagènes résultant d'un traitement de solubilisation au moins partielle dudit résidu insoluble.

Les étapes du procédé selon l'invention vont maintenant être décrites en détail :

La mise en forme des tissus placentaires (qu'il s'agisse de tissus frais ou du résidu final des tissus traités en vue de la préparation des gamma-globulines et de l'albumine thérapeutiques) consiste en une succession de pressages, broyages, lavages en milieu neutre et acide. Ce matériau est alors soumis à un premier traitement alcalin, par exemple par des solutions de soude à 0,5 M ou de chaux saturée pendant des temps variables à des températures ne dépassant pas 10 °C. Ce premier traitement est essentiel pour plusieurs raisons :

— Il élimine la majeure partie des composés non collagéniques qui sont solubilisés ;

— Il est une première étape d'inactivation des virus de l'hépatite ;

Il solubilise directement 10 à 20 % du collagène qui pourraient être purifiés par la méthode générale décrite par la suite (extrait 1) ;

— Il facilite la solubilisation ultérieure du collagène insoluble avec ou sans l'aide d'enzymes protéolytiques ;

— Il fournit un résidu insoluble A équivalent à seulement 20 % en poids du matériau sec de départ mais contenant plus de 70 % de collagène (> 80 % du collagène de départ).

A partir de ce résidu A, il est ensuite possible de préparer un matériau collagénique insoluble et de solubiliser 20 à 35 % du collagène total sans utilisation d'enzymes protéolytiques.

Pour ce faire, le résidu A est traité successivement par une seconde solution alcaline (par exemple solution de soude 0,1 à 0,5 M à 4 °C pendant 12 à 48 heures) (extrait 2) puis par une solution acide diluée (par exemple acide acétique (0,1 à 0,5 M) (extrait acide) à 4 °C pendant 5 à 12 heures). Le résidu est appelé Résidu B.

L'avantage de ce traitement du résidu A est multiple :

— inactivation complète des virus de l'hépatite ;

— préparation d'un matériau insoluble (résidu B) purifié en collagène (teneur en collagène > 85 %) et correspondant à 50-70 % du collagène de départ ;

— solubilisation de 20 à 35 % du collagène total sans enzymes.

Ces collagènes se trouvent concentrés dans le deuxième extrait alcalin (60-70 % de collagène) et dans l'extrait acide (75-85 % de collagène) ce qui facilite leur ultime purification.

A partir du résidu A, il est également possible de solubiliser la quasi-totalité des collagènes présents.

Le résidu A est soumis à une digestion enzymatique ménagée par des enzymes protéolytiques comme la pepsine, la pronase, la papaïne, la trypsine. Du fait de la fragilisation par le premier traitement alcalin des extrémités non hélicoïdales, les collagènes sont solubilisés en totalité. L'extrait déjà purifié en collagène (70-80 %) peut ensuite subir le protocole de fractionnement mis au point pour les différents extraits.

La purification et le fractionnement des collagènes présents dans les différents extraits (extraits alcalins n° 1 et 2, extrait acide et extrait enzymatique) sont réalisés selon le protocole général suivant : la première étape comporte une chromatographie sur résine échangeuse d'anions de type DEAE, à l'échelle industrielle à une température ne dépassant pas 10 °C. Les collagènes à l'état natif ne sont pas retenus sur la colonne alors que les glycoprotéines acides et les protéoglycannes contaminants restent sur la colonne. De plus, dans le cas de l'extrait alcalin n° 1 qui contient encore des virus de l'hépatite éventuellement non détruits, cette chromatographie permet la séparation des collagènes des contaminants viraux.

La seconde étape est la précipitation fractionnée des collagènes par des sels en milieu acide. Ainsi la fraction collagénique non retenue sur la colonne de DEAE-Sphérosil est concentrée puis passée en un milieu acide (acide acétique 0,1 M). Par addition progressive de sels neutres (ex. NaCl) les collagènes de Types I et III sont précipités à 0,7 M en NaCl puis les collagènes de types IV et V à 1,2 M NaCl. Si la séparation ultime de ces deux groupes de collagènes est nécessaire, il est alors possible d'utiliser des méthodes classiques. Pour les collagènes de type I et III une chromatographie sur DEAE-Sphérosil avec élution par gradient de force ionique croissante et pour les collagènes de Types IV et V une précipitation du Type V par une solution $Na_2HPO_4$ 0,02 M. Le collagène de Type IV restant en solution.

Les collagènes extraits du placenta par le procédé décrit ici sont chimiquement différents des collagènes extraits par attaque pepsique du placenta total. En effet, le traitement alcalin induit une désamidation partielle (20 à 30 %) de l'asparagine et de la glutamine qui se traduit par une charge négative plus importante. La solubilité en milieu neutre de ces collagènes est améliorée. Leurs dimensions moléculaires et leur structure hélicoïdale ne sont pas modifiées mais leur capacité à reformer des fibrilles in vitro est diminuée comme pour les collagènes pepsinés. Leurs propriétés biologiques ne sont cependant pas altérées ce qui permet leur utilisation comme biomatériaux comme décrit par la suite.

La présente invention va maintenant être illustrée par les exemples suivants donnés à titre non limitatif.

Exemple 1

Des placentas humains (160 kg humides) fraîchement prélevés et congelés, transportés à − 20 °C et décongelés par passage sur une vis chauffante, sont broyés et pressés de façon à séparer le sang intersticiel du tissu placentaire. Le tissu est alors lavé dans une solution de NaCl 9 % contenant de l'éthanol (3 %) à raison de quatre volumes de solution pour 1 volume de tissu, de façon à éliminer le maximum de liquide sanguin et d'hémoglobine.

Le tissu résiduel (80 kg humides contenant 14 % de matière sèche soit 11,2 kg dont 14,8 % de collagène soit 1,65 kg), est considéré comme le matériau de départ pour le calcul des taux d'extraction. Il est soumis à un premier traitement alcalin par 2 × 400 litres de solution de NaOH 0,5 M à 4 °C pendant 48 heures. Après centrifugation 30 min. à 2 000 g, l'extrait alcalin (n° 1) contenant 15 % du collagène de départ est soit éliminé, soit soumis au schéma de purification des collagènes décrits dans l'exemple n° 2. Le résidu du traitement alcalin n° 1 (20 kg humides) est appelé résidu A. Il contient 17 % du poids sec de départ soit 1,9 kg et 82 % du collagène de départ (1,35 kg), sa teneur en collagène étant en effet de 71 %.

Ce résidu A est soumis à un second traitement alcalin (100 litres de NaOH 0,5 M) pendant 48 heures à 4 °C. Après centrifugation l'extrait alcalin n° 2 (0,4 kg) qui contient 16 % du collagène total peut être soit éliminé, soit traité selon le protocole décrit dans l'exemple n° 2. Le résidu de ce deuxième traitement alcalin est extrait par 2 × 80 litres d'une solution acide acétique 0,5 M pendant 12 heures à 10 °C. Cet extrait acide contient 5 % du collagène total (0,08 kg). Le résidu final, appelé résidu B, est lavé à l'eau distillée puis conservé congelé ou lyophilisé suivant l'utilisation prévue. Il correspond à 12 % du poids sec de départ (1,34 kg) et à 68 % du collagène (1,13 kg). Sa teneur en collagène est en effet de 85 %.

Exemple 2

20 kg du résidu A (résidu du premier traitement alcalin du tissu placentaire) et contenant 1,9 kg de matière sèche dont 1,35 kg de collagène sont placés dans 100 litres d'acide acétique 0,5 M contenant 100 g de pepsine (Organotechnie, Type 700, 10 000 NS). Après incubation 24 heures à 10 °C, le résidu insoluble (moins de 10 % du résidu A) est éliminé par centrifugation. L'extrait pepsique est concentré par ultrafiltration sur membrane vendue sous la marque Millipore (PTGC 1000) puis repris et équilibré dans un tampon phosphate 0,1 M pH 6,8.

Cette solution est chromatographiée sur une colonne Pharmacia de 50 cm/100 cm garnie d'une résine DEAE vendue sous la marque Sphérosil à une vitesse d'élution de 10 l/heure. la fraction non retenue (80 l) qui contient les collagènes est ensuite de nouveau concentrée et équilibrée dans 50 litres d'acide acétique 0,5 M par ultrafiltration. Des quantités croissantes de NaCl sont ajoutées à cette solution et à une concentration de 0,7 M en NaCl les collagènes de Types I et III coprécipitent (720 g) et à 1,2 M en NaCl les collagènes de types IV et V (50 g). Afin d'améliorer la purification, on peut réaliser des redissolutions et des reprécipitations salines successives.

Ce processus de purification (chromatographie sur DEAE vendu sous la marque Sphérosil + précipitations salines) peut être utilisé pour les collagènes extraits selon l'exemple 1 sans digestion enzymatique et contenus dans les extraits alcalins n° 1 et n° 2 et l'extrait acide.

Le dosage immunologique par radioimmunoessai de l'antigénicité virale dans les différents résidus et extraits de ces deux exemples montre que :

— après deux traitements alcalins, l'antigène viral a complètement disparu ;

— après un seul traitement alcalin, l'antigénicité est atténuée mais encore présente. Elle est alors éliminée totalement des collagènes extraits par la chromatographie sur DEAE vendue sous la marque Sphérosil.

L'invention concerne également les applications industrielles, notamment dans les domaines de la biotechnologie et de la pharmacie, des différentes préparations de collagènes placentaires humains obtenus par mise en œuvre du procédé selon l'invention.

Applications en biotechnologie :

a) Cultures de cellules :

Les collagènes obtenus selon l'invention sont utilisables en tant que supports de culture in vitro pour diverses espèces et types cellulaires différenciés.

A titre d'exemple non limitatif, on peut indiquer la culture cellulaire suivante :

Les collagènes insolubles (résidu B de l'exemple 1) obtenus à la suite des extractions alcalines (étapes 1 et 2) et constitués des mélanges des types I, III, V, sont texturés sous forme de film puis déposés sur le fond de différents récipients pour culture de tissu tels que cuves, flacons, boîtes de Pétri, etc... De même, les collagènes placentaires solubles de différents types obtenus selon le procédé objet de l'invention peuvent être déposés avec ou sans réticulation à la surface de divers récipients (flacons, boîtes, lamelles, etc...) ou matériaux (billes de verre, etc...) et servir de supports de culture à divers types cellulaires. Les récipients contenant la couche de collagène sont ensuite stérilisés soit à la chaleur (110 °C à l'autoclave pendant 40 min.) soit par rayonnements (rayons γ ou ultraviolets). On peut alors procéder à la mise en culture d'hépatocytes de la manière suivante : on procède à l'exérèse chirurgicale stérile d'un fragment de foie de singe. Le prélèvement est sectionné en petits fragments de 5 mm de diamètre, placés en milieu de Eagle puis dissociés par grattage entre 2 lames de verre stériles. La suspension cellulaire obtenue est dénombrée par dilution-numération en cellule de Thoma sous le microscope, puis ajustée à $10^7$ cellules/ml en milieu Eagle additionné de 10 % de sérum de veau fœtal. On dépose alors 10 ml ($100 \times 10^6$ cellules) de la suspension cellulaire sur le film de collagène contenu dans un flacon de 250 ml et on rajoute 90 ml de milieu de Eagle additionné de 10 % de sérum de veau fœtal. Le flacon fermé est placé à l'étuve à 37 °C pendant 24 heures. Le surnageant liquide est alors éliminé puis remplacé par 100 ml de milieu neuf (Eagle + 20 % sérum veau fœtal). Le flacon est replacé à 37 °C pendant plusieurs jours. Dans ces conditions, la culture d'hépatocytes différenciés sur lit de collagène peut être observée pendant plus de 10 jours. Cette culture d'hépatocytes diférenciés peut constituer un support de replication et de multiplication des virus de l'hépatite humaine en vue de la fabrication ultérieure d'un vaccin.

Les cultures de diverses cellules différenciées telles que lymphocytes, macrophages, chondrocytes, endothéliocites, cellules nerveuses, cellules glandulaires peuvent être également obtenues sur couche de collagène placentaire humain.

b) Culture d'embryons :

Les boîtes de Pétri recouvertes d'un gel de collagène insoluble peuvent recevoir des ovocytes fécondés de mammifères, cultivés en milieu de Ham. Le support collagénique facilite une meilleure nidation et une segmentation améliorée et prolongée de l'ovocyte au-delà du stade blastocyste. Cette culture embryonnaire avancée autorise une observation, une analyse et une sélection accrues de l'embryon avant son transfert dans l'utérus.

c) Culture de tissus ou d'organes :

La culture de fragments de tissus ou d'organes différenciés (peau, glandes thyroïde, surrénale, pancréas, etc...) peut également être obtenue sur supports collagéniques placentaires humains obtenus selon l'invention.

d) Fabrication de matériels et appareils utilisables en génie biologique et médical et en génie industriel :

Les collagènes obtenus selon l'invention sont utilisables par exemple pour la fabrication d'appareils à visées thérapeutiques d'épuration ou de complémentation du plasma ou du sang humain (dialyse, pancréas artificiel, etc...). C'est ainsi qu'ils peuvent être utilisés sous forme de membranes pouvant être montées en parallèle dans un module destiné à la dialyse extracorporelle chez l'homme. Les membranes peuvent être prétraitées avant montage, par exemple par greffage ou inclusion dans ou sur le matériel collagénique de molécules biologiques actives : anticorps, antigènes, enzymes, hormones, etc..., de façon à présenter une spécificité d'action immunologique, enzymatique ou hormonale qui se manifestera lors d'une dialyse, d'une épuration ou d'une complémentation thérapeutique par circulation sanguine extracorporelle chez l'homme.

De même, les collagènes obtenus selon l'invention et inclus sous forme de membranes dans des appareils industriels de filtration, de dialyse ou de fractionnement, permettent d'obtenir des séparations industrielles de liquides biologiques complexes hautement résolutives par chromatographie d'affinité.

Fabrication de prothèses et de biomatériaux à usage pharmaceutique

a) Collagènes insolubles :

Les collagènes insolubles selon l'invention peuvent être utilisés dans la fabrication d'éponges hémostatiques ou encore texturés sous forme de fils de suture, ou de membranes à usage chirurgical.

Les collagènes insolubles obtenus selon l'invention peuvent être texturés en film pouvant recevoir par codéposition, par adsorption, par emprisonnement mécanique ou par greffage tous les composés biologiquement actifs tels qu'enzymes, hormones, antigènes, anticorps, vitamines et facteurs de croissance ; ces films de collagène ainsi activés constituent des prothèses implantables biologiquement actives et aux effets thérapeutiques permanents et filés pour un certain nombre d'affections humaines.

b) Collagènes solubles :

Les collagènes solubles préparés sous forme de microfibrilles biodégradables sont utilisables soit isolément dans les thérapeutiques de complémentation post-traumatique, soit combinés à de l'hémoglobine humaine en vue par exemple de la fabrication de sang synthétique homologue biodégradable.

Les prothèses et biomatériaux préparés à partir des différents types de collagènes humains d'origine placentaire, seuls ou en association, obtenus selon l'invention, sont dépourvus d'antigénicité notable pour l'espèce humaine. Ils sont de ce fait bien tolérés et représentent une nouvelle génération de biomatériaux pour la médecine humaine.

## Revendications

1. Procédé de préparation industrielle de matériaux collagéniques à partir de tissus placentaires, comportant une étape de prétraitement desdits tissus, un traitement en milieu alcalin desdits tissus et un traitement éventuel de digestion de résidus insolubles, issus du traitement alcalin, caractérisé en ce qu'on part de tissus placentaires humains, on prétraite ces tissus par pressage, broyage et au moins un lavage en milieu neutre ou acide, on soumet les tissus ainsi prétraités à un milieu fortement alcalin à une température inférieure ou égale à 10 °C jusqu'à obtention de portions de collagène solubilisées et d'un résidu insoluble, et on soumet à une chromatographie sur résine échangeuse d'anions, à une température inférieure ou égale à 10 °C suivie d'une précipitation fractionnée des collagènes par des sels en milieu acide, lesdites portions de collagène solubilisées ainsi que, éventuellement, les solutions de collagène résultant d'un traitement de solubilisation au moins partielle dudit résidu insoluble.

2. Procédé selon la revendication 1, caractérisé en ce que les tissus placentaires humains sont choisis parmi les tissus frais et les résidus tissulaires finals de placentas traités en vue de la préparation des gamma-globulines et de l'albumine thérapeutique.

3. Procédé selon les revendications 1 et 2, caractérisé en ce que le milieu fortement alcalin est choisi parmi les solutions de soude et de potasse à 0,5 M et la chaux saturée.

4. Procédé selon la revendication 1, caractérisé en ce que le traitement de solubilisation du résidu insoluble est un traitement en milieu alcalin à température inférieure à 10 °C et de préférence aux environs de 4 °C, éventuellement suivi d'un traitement acide à une température inférieure à 10 °C et de préférence aux environs de 4 °C.

5. Procédé selon l'une quelconque des revendications 1 à 4, caractérisé en ce que le résidu insoluble est solubilisé par digestion enzymatique ménagée par des enzymes protéolytiques.

6. Procédé selon l'une quelconque des revendications 1 à 4, caractérisé en ce que les résidus du collagène insolubles provenant des différentes étapes du traitement sont isolés par toute méthode connue en soi.

7. Matériaux collagéniques obtenus directement par mise en œuvre du procédé selon l'une quelconque des revendications 1 à 6.

8. Applications des matériaux collagéniques selon la revendication 7 comme biomatériaux utilisables en biotechnologie et en pharmacie.

9. Applications des matériaux collagéniques selon la revendication 7 comme supports de culture de cellules, d'embryons, de tissus et/ou d'organes.

10. Applications des matériaux collagéniques selon la revendication 7 en tant qu'éponges à usage hémostatique, fils de suture, solutés et supports biodégradables de composés biologiquement actifs et prothèses.

11. Applications des matériaux collagéniques selon la revendication 7 en tant que matériels et appareils extracorporels utilisables en génie biomédical pour des thérapeutiques d'épuration ou de complémentation du plasma ou du sang humain après greffage ou inclusion dans ou sur le matériel collagénique de molécules biologiques actives : anticorps, antigènes, enzymes, hormones.

12. Applications des matériaux collagéniques selon la revendication 7 en tant que matériels et appareils utilisables en génie industriel pour les processus de fractionnement sélectifs de liquides biologiques complexes.

## Claims

1. A process for the industrial preparation of collagenous materials from placental tissues, comprising a pre-treatment stage of the said tissues, a treatment of the said tissues in an alkaline medium and possibly a digestion treatment of the insoluble residues from the alkaline treatment, characterized in that starting with human placental tissue these tissues are pre-treated by pressing, pulverizing, and washing at least once in a neutral or acid medium, the tissues after pre-treatment are subjected to a strongly alkaline medium at a temperature of 10 °C or below until portions of collagen rendered soluble and an insoluble residue are obtained, and the said portions of collagen rendered soluble as well as, possibly, collagen solutions resulting from a treatment of the said insoluble residue to render it at least partially soluble are treated by anion resin exchange chromatography at a temperature of 10 °C or below followed by fractional precipitation of the collagen by salts in an acid medium.

2. A process according to Claim 1, characterized in that the human placental tissues are selected from fresh tissues and the final tissue residues of placentas that have been treated for the purpose of preparing gamma globulins and therapeutic albumen.

3. A process according to Claims 1 and 2, characterized in that the strongly alkaline medium is selected from amongst 0.5 M solutions of caustic soda and potash and saturated limewater.

4. A process according to Claim 1, characterized in that the treatment ot make the insoluble residue soluble is a treatment in alkaline medium at a temperature less than 10 °C and preferably about 4 °C, possibly followed by an acid treatment at a temperature less than 10 °C and preferably about 4 °C.

5. A process according to any of Claims 1 to 4, characterized in that the insoluble residue is rendered soluble by enzyme digestion provided by proteolytic enzymes.

6. A process according to any of Claims 1 to 4, characterized in that the insoluble collagen residues arising from the different stages of the treatment are separated by any method that in itself is known.

7. Collagenous materials directly obtained by operation of the process according to any of claims 1 to 6.

8. Application of the collagenous materials according to claim 7 as biomaterials that can be used in biotechnology and in pharmacy.

9. Applications of the collagenous materials according to Claim 7 as supports for the culture of cells, embryos, tissues and/or organs.

10. Applications of the collagenous materials according to Claim 7 in the form of sponges for haemostatic use, suture threads, biodegradable aqueous solutions and supports of biologically active compounds and prostheses.

11. Applications of the collagenous materials according to Claim 7 in the form of material and equipment that can be used outside the body in biomedical engineering for the therapies of purification or complementation of plasma or human blood after skin grafting, or inclusion in or on the collagenous material of biologically active molecules : antibodies, antigens, enzymes or hormones.

12. Applications of the collagenous materials according to Claim 7 in the form of materials and equipment that can be used in industrial engineering for processes of selective fractionating of complex biological liquids.

**Patentansprüche**

1. Verfahren zur industriellen Herstellung von Kollagenmaterialien ausgehend von plazentaren Geweben, das einen Schritt der Vorbehandlung der Gewebe, eine Behandlung der Gewebe im alkalischen Milieu und gegebenenfalls eine Behandlung zur Digestion unlöslicher, von der alkalischen Behandlung herrührender Rückstände umfaßt, dadurch gekennzeichnet, daß von plazentaren Humangeweben ausgegangen wird, diese Gewebe durch Pressen, Zerkleinern und zumindest eine Wäsche im neutralen oder sauren Milieu vorbehandelt werden, die so vorbehandelten Gewebe bei einer Temperatur, die niedriger als oder gleich 10 °C ist, einem stark alkalischen Milieu bis zur Gewinnung von solubilisierten Kollagenmengen und von einem unlöslichen Rückstand unterworfen werden und die solubilisierten Kollagenmengen sowie gegebenenfalls die Kollagenlösungen, die von einer Behandlung mit zumindest teilweiser Solubilisierung des unlöslichen Rückstandes herrühren, einer chromatographie mit Anionenaustauscherharz, einer Temperature, die niedriger als oder gleich 10 °C ist, gefolgt von einer fraktionierten Fällung der Kollagene durch Salze im sauren Milieu unterworfen werden.

2. Verfahren nach Anspruch 1, dadurch gekennzeichnet, daß die plazentaren Humangewebe unter den frischen Geweben und den Gewebeendrückständen von Plazentas ausgewählt werden, die in Hinsicht auf die Gewinnung von Gammaglobulinen und von therapeutischem Albumin behandelt worden sind.

3. Verfahren nach den Ansprüchen 1 und 2, dadurch gekennzeichnet, daß das stark alkalische Milieu unter den Lösungen von kaustische Soda und Kali mit 0,5 M und gesättigtem Kalk ausgewählt wird.

4. Verfahren nach Anspruch 1, dadurch gekennzeichnet, daß die solubilisierungsbehandlung des unlöslichen Rückstandes eine Behandlung im alkalischen Milieu bei einer Temperatur unterhalb von 10 °C und vorzugsweise in der Umgebung von 4 °C, gegebenenfalls gefolgt von einer sauren Behandlung bei einer Temperatur unterhalb von 10 °C und vorzugsweise in der Umgebung von 4 °C, ist.

5. Verfahren nach einem beliebigen der Ansprüche 1 bis 4, dadurch gekennzeichnet, daß der unlösliche Rest durch enzymatische Digestion solubilisiert wird, die durch proteolytische Enzyme herbeigeführt wird.

6. Verfahren nach einem beliebigen der Ansprüche 1 bis 4, dadurch gekennzeichnet, daß die unlöslichen Kollagenrückstände, die von den verschiedenen Behandlungsschritten herrühren, durch jede an sich bekannte methode isoliert werden.

7. Kollagenmaterialien, die direkt durch Ausführung des Verfahrens nach einem beliebigen der Ansprüche 1 bis 6 erhalten worden sind.

8. Verwendungen der Kollagenmaterialien nach Anspruch 7 als in der Biotechnologie und in der Pharmazie verwendbare Biomaterialien.

9. Verwendungen der Kollagenmaterialien nach Anspruch 7 als Kulturträger für Zellen, Embryonen, Gewebe und/oder Organe.

10. Verwendungen von Kollagenmaterialien nach Anspruch 7 als Schwämme zum hämostatischen Gebrauch, Nähfäden, biologisch abbaubare gelöste Stoffe und Träger aus biologisch aktiven Verbindungen und Prothesen.

11. Verwendungen von Kollagenmaterialien nach Anspruch 7 als Materialien und Vorrichtungen außerhalb des Körpers, die in der biomedizinischen Technologie für die Reinigungstherapeutiken oder die Komplementierung von Plasma oder von menschlichem Blut nach einpflanzung/Impfen oder Einschluß in oder auf dem Kollagenmaterial von biologisch aktiven Molekülen : Antikörpern, Antigenen, Enzymen, Hormonen, verwendbar sind.

12. Verwendungen der Kollagenmaterialien nach Anspruch 7 als Materialien und Vorrichtungen, die in der industriellen Technik für die selektiven Fraktionierungsprozesse von biologischen komplexen Flüssigkeiten verwendbar sind.